# EUROPEAN PATENT SPECIFICATION

(11) **EP 2 061 530 B1**
(45) Date of publication and mention of the grant of the patent: **26.02.2014**
(21) Application number: 07836145.8
(22) Date of filing: 18.07.2007
(51) Int. Cl.: A61L 31/16

(54) **IMPLANTABLE DEVICES CONTAINING NUCLEAR RECEPTOR LIGANDS FOR THE TREATMENT OF VASCULAR AND RELATED DISORDERS**
IMPLANTIERBARE VORRICHTUNGEN MIT NUKLEAREN REZEPTORLIGANDEN ZUR BEHANDLUNG VON GEFÄSSERKRANKUNGEN UND ASSOZIIERTEN ERKRANKUNGEN
DISPOSITIFS IMPLANTABLES CONTENANT DES LIGANDS DE RÉCEPTEUR NUCLÉAIRE DESTINÉS AU TRAITEMENT DE TROUBLES VASCULAIRES ET D'AUTRES TROUBLES ASSOCIÉS

(30) Priority: 19.07.2006 US 832059 P; 19.07.2006 US 832173 P; 20.07.2006 US 832339 P; 17.07.2007 US 879297
(43) Date of publication of application: 27.05.2009
(73) Proprietor: Abbott Cardiovascular Systems Inc., Santa Clara, CA 95054-2807 (US)
(72) Inventor: CONSIGNY, Paul, San Jose, California 95111 (US); CHENG, Jin, Livermore, California 94550 (US); ROORDA, Wouter Erik, Palo Alto, California 94306 (US)
(74) Representative: ZBM Patents
(86) International application number: PCT/US2007/016351
(87) International publication number: WO 2008/011093

(56) References cited:
- EP-A- 1 759 724
- WO-A-03/024473
- WO-A-03/035132
- WO-A1-03/075911
- WO-A1-2005/113519
- WO-A2-2004/066963
- US-A1- 2004 102 373
- US-B1- 6 455 062

## Description

### BACKGROUND OF THE INVENTION

### Field of the Invention

The present invention is directed to implantable devices (e.g., drug-delivery stents) containing nuclear receptor ligands and the use of such devices in treating or preventing vascular disorders (e.g., restenosis) and related disorders.

### Description of the State of the Art

Angioplasty is a well-known procedure for treating heart disease. A problem associated with angioplasty includes formation of intimal flaps or tom arterial linings which can collapse and occlude the conduit after the balloon is deflated. Moreover, thrombosis and restenosis of the artery may develop over several months after angioplasty, which may require another angioplasty procedure or a surgical by-pass operation. "Stenosis" refers to a narrowing or constriction of the diameter of a bodily passage or orifice, and "restenosis" refers to the reoccurrence of stenosis in a blood vessel or heart valve after it has been treated (as by balloon angioplasty, stenting, or valvuloplasty) with apparent success.

Stents are often used in the treatment of atherosclerotic stenoses in blood vessels. To reduce the partial or total occlusion of the artery by the collapse of arterial lining and to reduce the chance of thrombosis and restenosis following angioplasty in the vascular system, a stent may be implanted in the lumen to reinforce body vessels and maintain the vascular patency. A "lumen" refers to a cavity of a tubular organ such as a blood vessel. As a mechanical intervention, stents act as scaffoldings, functioning to physically hold open and, if desired, to expand the wall of a passageway, e.g., a blood vessel, urinary tract or bile duct.

Stents are also used as a vehicle for providing biological therapy. Biological therapy can be achieved by medicating the stents. Medicated stents provide for the local administration of a therapeutic substance at the diseased site, thereby possibly avoiding side effects associated with systemic administration of such medication. One method of medicating stents involves the use of a polymeric carrier coated onto the surface of a stent, where a therapeutic substance is attached to, blended with, or impregnated in the polymeric carrier.

US6455062 describes an implantable medical device, i.e. a stent, comprising a retinoid for improving biocompatibility and a therapeutic agent. The bioactive substance is sustained released.

WO03035132 refers to a drug delivery system comprising a stent with a polymeric coating and a biological agent selected from the group of retinoic acid, Matrigel, laminin and laminin derived peptides.

WO03/024473 discloses methods and compositions for inducing or enhancing chondrogenesis in vivo/ or ex vivo by using RAR pan-antagonist compositions for the treatment, repair and engineering of cartilage.

US2004/102373 discloses a composition as a method for increasing the progenitor cell population in a mammal comprising a biocompatible acellular matrix made of collagen and a specific amino acid and protein.

WO03/07591 refers to the use of PPAR agonists for the treatment of vascular disease such as atherosclerosis, hypertension restenosis and renal diseases, etc. The PPAR agonists are selected from the group consisting of clofibrate, benzafibrate, fenofibrate, etc. The PPAR agonist may be contained within or adapted to be released by a surgical or medical device, e.g. stent, catheter, prostheses, sutures and the like.

W02005/113519 discloses compounds and compositions as PPAR modulator as well as methods of using such compounds to treat or prevent diseases or disorders associated with the activity of the Peroxisome Proliferator-Activated Receptor families.

WO2004/066963 discloses a class of derivatives which are agonists or partial agonists or antagonists of PPAR gamma and are useful in the treatment and control of hyperglycemia, but also restenosis etc.

The present invention is directed to implantable devices containing nuclear receptor ligands useful for treating or preventing vascular or related disorders. The therapeutic agents may be used alone or in combination. The implantable devices include drug-delivery stents that locally deliver anti-thrombotic and anti-restenosis agents to the site of implantation.

### SUMMARY OF THE INVENTION

The present invention is directed to an implantable device formed of a material comprising at least one biologically active ("bioactive") agent selected from nuclear receptor ligands, nuclear receptor agonists and nuclear receptor antagonists that affect local gene expression or transcription at the site of implantation of the device. The at least one bioactive agent includes at least one peroxisome proliferator-activated receptor (PPAR) ligand, PPAR agonist or PPAR antagonist as mentioned in claim 1.

In another embodiment, the at least one bioactive agent includes at least one synthetic or natural retinoid. In a specific embodiment, the at least one synthetic or natural retinoid includes tazarotene.

The at least one bioactive agent includes at least one nuclear receptor ligand selected from the group consisting of thiazolidinethiones, glitazone, troglitazone, pioglitazone, rosiglitazone.

In some embodiments, the at least one bioactive agent is embedded in micropores or nanopores on and/or in the implantable device. In other embodiments, the implantable device itself is made of the material comprising the at least one bioactive agent. In still other embodiments, the material comprising the at least one bioactive agent is a coating disposed over at least a portion of the implantable device.

In one embodiment, the implantable device has a bioactive agent-release profile that includes one or a combination of a pulse, burst and sustained release profile. In certain embodiments, the implantable device provides sustained release of the at least one bioactive agent over a period up to 24 months, or up to 6 months, or up to 3 months. In a further embodiment, the implantable device is capable of simultaneously releasing two or more bioactive agents.

In an embodiment, the implantable device is a stent, graft, stent-graft, catheter, lead or electrode, clip, shunt, closure device, valve, particle (e.g., bioactive-eluting particle), microparticle, nanoparticle, gel or emulsion. In a specific embodiment, the device is a stent.

The implantable device is for use in the treatment or prevention of a condition or disorder selected from atherosclerosis, thrombosis, restenosis, hemorrhage, vascular dissection, vascular perforation, vascular aneurysm, vulnerable plaque, chronic total occlusion, patent foramen ovale, claudication, anastomotic proliferation for vein and artificial grafts, arteriovenous anastamoses, bile duct obstruction, ureter obstruction and tumor obstruction. In a particular embodiment, the device is for use in the treatment or prevention of atherosclerosis, thrombosis, restenosis or vulnerable plaque.

Further embodiments of the disclosure are drawn to the use of at least one biologically active agent selected from nuclear receptor ligands, nuclear receptor agonists and nuclear receptor antagonists in the manufacture of an implantable device, wherein:
the nuclear receptor ligands, agonists and antagonists affect local gene expression or transcription at the site of device implantation, and
the device is for use in the treatment or prevention of a condition or disorder selected from the group consisting of atherosclerosis, thrombosis, restenosis, hemorrhage, vascular dissection, vascular perforation, vascular aneurysm, vulnerable plaque, chronic total occlusion, patent foramen ovale, claudication, anastomotic proliferation for vein and artificial grafts, arteriovenous anastamoses, bile duct obstruction, ureter obstruction and tumor obstruction.

In specific embodiments of the invention, the implantable device is useful for the treatment or prevention of atherosclerosis, thrombosis, restenosis or vulnerable plaque.

Various embodiments of the invention are described in further detail below.

### BRIEF DESCRIPTION OF THE DRAWINGS

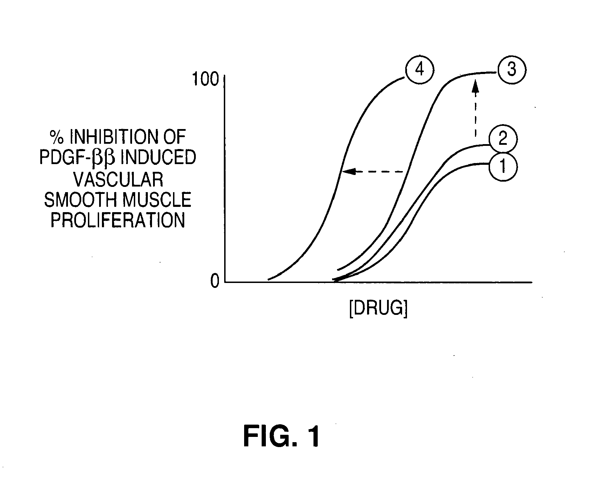

Figure 1 shows expected results for the inhibition of vascular smooth muscle cell proliferation in response to a mitogen such as PDGF. Curve 1 is for drug 1, curve 2 is for drug 2, and curves 3 and 4 are for different combinations of drugs.

### DETAILED DESCRIPTION OF THE INVENTION

### Terms and Definitions

The following definitions apply:

The terms "biologically degradable" (or "biodegradable"), "biologically erodable" (or "bioerodable"), "biologically absorbable" (or "bioabsorbable"), and "biologically resorbable" (or "bioresorbable"), in reference to polymers and coatings, are used interchangeably and refer to polymers and coatings that are capable of being completely or substantially completely degraded, dissolved, and/or eroded over time when exposed to bodily fluids such as blood and can be gradually resorbed, absorbed and/or eliminated by the body, or that can be degraded into fragments that can pass through the kidney membrane of an animal (e.g., a human), e.g., fragments having a molecular weight of about 40,000 Daltons (40 kDa) or less. The process of breaking down and eventual absorption and elimination of the polymer or coating can be caused by, e.g., hydrolysis, metabolic processes, oxidation, enzymatic processes, bulk or surface erosion, and the like. Conversely, a "biostable" polymer or coating refers to a polymer or coating that is not biodegradable.

Whenever the reference is made to "biologically degradable," "biologically erodable," "biologically absorbable," and "biologically resorbable" stent coatings or polymers forming such stent coatings, it is understood that after the process of degradation, erosion, absorption, and/or resorption has been completed or substantially completed, no coating or substantially little coating will remain on the stent. Whenever the terms "degradable," "biodegradable," or "biologically degradable" are used in this application, they are intended to broadly include biologically degradable, biologically erodable, biologically absorbable, and biologically resorbable polymers or coatings.

"Physiological conditions" refer to conditions to which an implant is exposed within the body of an animal (e.g., a human). Physiological conditions include, but are not limited to, human body temperature (approximately 37 °C) and an aqueous environment of physiologic ionic strength, pH and enzymes.

As used herein, a "ligand" is a chemical or biological substance that is able to bind to a substrate. The ligand may be a full or partial agonist, antagonist, activator and/or repressor of that substrate.

As used herein, a material that is described as a coating "disposed over" an indicated substrate, e.g., an implantable device, refers to a coating of the material deposited directly or indirectly over at least a portion of the surface of the substrate. Direct depositing means that the coating is applied directly to the exposed surface of the substrate. Indirect depositing means that the coating is applied to an intervening layer that has been deposited directly or indirectly over the substrate.

As used herein, an "implantable device" may be any device that can be implanted in an animal. Examples of implantable devices include, but are not limited to, self-expandable stents, balloon-expandable stents, coronary stents, peripheral stents, stentgrafts, catheters, other expandable tubular devices for various bodily lumen or orifices, grafts, vascular grafts, arterio-venous grafts, by-pass grafts, pacemakers and defibrillators, leads and electrodes for the preceding, artificial heart valves, anastomotic clips, arterial closure devices, patent foramen ovale closure devices, cerebrospinal fluid shunts, particles (e.g., bioactive-eluting particles), microparticles, nanoparticles, gels, and emulsions. As an example, stents may be intended for any vessel in the body, including neurological, carotid, vein graft, coronary, aortic, renal, iliac, femoral, popliteal vasculature, and urethral passages. Further, stents can be of virtually any design, and can be made of a metallic material, an alloy, a polymeric material, or a combination thereof.

An implantable device can be designed for the localized delivery of a therapeutic agent. A medicated implantable device may be constructed in part, e.g., by coating the device with a coating material containing a therapeutic agent. The body of the device may also contain a therapeutic agent.

An implantable device can be fabricated with a coating containing partially or completely a biodegradable/bioabsorbable/ bioerodable polymer, a biostable polymer, or a combination thereof. An implantable device itself can also be fabricated partially or completely from a biodegradable/bioabsorbable/ bioerodable polymer, a biostable polymer, or a combination thereof.

### Embodiments of the Invention

The present invention is directed to an implantable device formed of a material comprising at least one biologically active ("bioactive") agent selected from nuclear receptor ligands, nuclear receptor agonists and nuclear receptor antagonists that affect local gene expression or transcription at the site of implantation of the device. The group of nuclear receptor ligands, nuclear receptor agonists and nuclear receptor antagonists includes, but is not limited to, peroxisome proliferator-activated receptor (PPAR) ligands, PPAR agonists, PPAR antagonists, and synthetic and natural retinoids. The invention is also directed to a method of treating or preventing vascular disorders and related disorders by use of the implantable device of the invention. Embodiments of the invention relating to nuclear receptor ligands, PPAR ligands and retinoids are described below.

### Nuclear Receptor Ligands, Agonists and Antagonists

Restenosis after balloon angioplasty or stent placement is the result of intimal thickening secondary to smooth muscle cell migration and proliferation, matrix deposition, and inappropriate vascular remodeling secondary to arterial shrinkage or a failure to expand. Restenosis is promoted by growth factors (e.g., PDGF, *β*FGF and IGF-1) and wall stresses that activate intracellular pathways (e.g., MAP kinase), resulting in the formation of transcription factors (e.g., AP-1). As an example, AP-1, is a c-fos/c-jun homodimer or heterodimer that induces gene transcription of factors involved in cell migration (e.g., collangenase and stromolysin) and cell proliferation (e.g., cell cycle factors). At the same time, inflammatory signals (e.g., TNF-*α* and IL-1*β*) bind to cell receptors and activate the NF-k*β* pathway that results in the transcription of proinflammatory cell ligands (e.g., ICAMs and ELAMs) and cytokines (e.g., IL-1*β*, IL-2 and IL-6).

These restenosis processes can be inhibited by using agonists or antagonists that are ligands for specific nuclear receptors. Such receptors and ligands include thiazolidinethiones, glitazone, troglitazone, pioglitazone, rosiglitazone.

Previous studies have demonstrated that individual ligands or hormones can be used to inhibit smooth muscle cell migration and proliferation and matrix synthesis. With regard to estrogens, clinically used estrogen inhibited human vascular smooth muscle cell proliferation. Dubey, Arterioscl. Thromb. Vasc. Biol., 20: 964-972 (2000). Moreover, estradiol inhibited proliferation and migration of human female vascular smooth muscle cells. Suzuki et al., Cardiovasc. Res., 32: 516-523 (1996).

Studies involving corticosteroids have shown that corticosteroids inhibited proliferation of smooth muscle cells isolated from human atherosclerotic arteries. Voisard et al., Int. J. Cardiol., 43: 257 (1994). One study showed that local periadventitial delivery of dexamethasone prevented neointimal thickening/proliferation after balloon injury of rat carotid arteries. Villa et al., J. Clin. Invest., 93: 1243-1249 (1994). Another study showed that dexamethasone suppressed intimal thickening/hyperplasia of rabbit carotid arteries after Fogarty balloon denudation. Chervu et al., J. Vasc. Surg., 10: 128-134 (1989).

A new mode of action recently has been attributed to nuclear receptors-transcriptional crosstalks, which indicates that nuclear receptors can compete with transcription factors such as AP-1, resulting in inhibition of transcription factor effectiveness. M. Gottlichel, J. Mol. Med., 76: 480-489 (1998). Examples of such crosstalk include glucocorticoid blocking AP-1 activity. C. Jonet, Cell, 62: 1189-2104 (1990). Further, an interleukin stimulated, and *all-trans* retinoic acid inhibited, collagenase gene expression at AP-1 binding sites. R. Llafyatis et al., Mod. Endocrinol., 4: 973-980 (1990). Retinoic acid also interfered with AP-1 binding and stromelysin gene expression. R. Nicholson et al., EMBO, 9: 4443-4454 (1990).

The present invention provides for the use of nuclear receptor ligands for the treatment or prevention of vascular disorders and related disorders. Such disorders include, but are not limited to, atherosclerosis, thrombosis, restenosis, hemorrhage, vascular dissection, vascular perforation, vascular aneurysm, vulnerable plaque, chronic total occlusion, patent foramen ovale, claudication, anastomotic proliferation for vein and artificial grafts, arteriovenous anastamoses, bile duct obstruction, ureter obstruction and tumor obstruction.

The nuclear receptor ligands, i.e., agonists, antagonists, activators and/or repressors of nuclear receptors, can modulate local gene expression or transcription at the site of delivery.

For example, nuclear receptor ligands can be used to treat or prevent restenosis after balloon angioplasty or vascular graft placement. The nuclear receptor ligands are expected to interfere with the gene expression or transcription that occurs after balloon angioplasty or stent deployment and that would naturally lead to restenosis.

The present invention encompasses both systemic and local administration of the nuclear receptor ligands. In one embodiment, the ligands are administered systemically. In another embodiment, the ligands are administered locally. More localized effects may be obtained by delivering the ligands to the diseased site locally using implantable devices (e.g., a local delivery catheter or a drug-delivery stent formed of a material (e.g., a polymeric coating) containing the ligands).

Individual nuclear receptor ligands can be used to obtain a beneficial therapeutic or prophylactic effect. However, a more beneficial effect, i.e., an additive or synergistic effect, may be obtained by using multiple nuclear receptor ligands that bind to different receptors. In this way, multiple inhibitor signals are sent to the target cell, resulting in targeted inhibition of gene expression or transcription.

Accordingly, the present invention also provides for a cocktail mixture of two or more nuclear receptor ligands that can be used to produce a greater inhibition of the disorder (e.g., restenosis) than that produced by a single ligand. This additive or synergistic effect can be used to inhibit intimal thickening and promote positive geometric enlargement, thereby inhibiting restenosis. The mixture can be composed of any combination of drugs, such as *all-trans* retinoic acid, 17-β estradiol, clobetasol and/or a glitazone. The exact mixture can be determined by *in vitro* and/or *in vivo* experiments, which would measure specific endpoints and allow the determination of the mixtures that produce the most desirable results.

**Figure 1** depicts expected results for the inhibition of vascular smooth muscle cell proliferation in response to a mitogen such as PDGF, using various nuclear receptor ligands. Curve 1 represents the results if drug 1 is used to protect against the effects of PDGF, and curve 2 represents the results if drug 2 is used. Curves 3 and 4 represent the results if different combinations of drugs are used, resulting in additive/synergistic effects. Alternative experiments could examine smooth muscle cell migration, smooth muscle cell collagen synthesis, and smooth muscle cell gene expression (e.g., MCP-1 gene expression, NF-*κβ* reporter gene expression and AP-1 reporter gene expression) after stimulation with a growth factor or cytokine.

### PPAR Ligands, Agonists and Antagonists

Peroxisome proliferator-activated receptors (PPARs) belong to the nuclear receptor superfamily. There are three subtypes of PPARs: PPAR-α, PPAR-*β*/*δ* and PPAR-γ. The PPARs modulate, i.e., activate or repress, gene expression upon activation of the PPARs by their ligands. Many naturally occurring PPAR ligands have been identified, and many synthetic PPAR ligands have been created. Ligands for PPAR-γ, both thiazolidinediones (TZD) and non-TZD compounds, were developed as insulin sensitizers to treat diabetes. Ligands for PPAR-α were developed as hypolipidemic agents.

PPARs mediated by their agonists have been reported to have the following effects, among others:
- PPAR-α and PPAR-γ agonists inhibit proliferation and migration of smooth muscle cells (SMC), which are important elements in arteriosclerosis, atherogenesis and restenosis after vascular intervention.
- PPAR-α and PPAR-γ agonists upregulate lipoprotein lipase expression, which could result in the treatment and prevention of arteriosclerosis and restenosis.
- All three PPARs regulate macrophage cholesterol homeostasis.
- PPAR-α and PPAR-γ agonists inhibit endothelial cell migration.
- PPAR-α strongly suppresses histocompatibility complex class II (i.e., MHC-II) expression and inhibits T-lymphocyte activation. T-lymphocytes are considered as atheroma-associated cells.
- PPAR-α and PPAR-γ agonists show profound anti-inflammatory effects, which are believed to be involved in restenosis.
- PPAR-α suppresses Il-6-induced C-reactive protein (CRP), which has been suggested to be associated with restenosis.
- PPAR-α agonists and PPAR-γ agonists strongly and moderately, respectively, inhibit the chemokine, monocyte chemoattractant protein-1 (MCP-1), which is induced by CRP.
- PPAR-γ agonists upregulate nitric oxide synthesis in vascular SMC.

In addition to the above effects, there is increasing evidence that PPARs are also involved in the following:
- Both PPAR-α and PPAR-γ. act to inhibit the activation of inflammatory response genes by negatively interfering with NF-*κβ*.
- Receptor overexpression of PPAR-β/δ increases cell proliferation.
- PPAR-α activators suppress IL-1-induced CRP, which is a nonspecific marker of inflammation.

PPAR-*δ* is a less understood subtype of the PPARs. It may be involved in vascular lesions, since overexpression of PPAR-*δ* increases cell proliferation. PPAR-*δ* may also play a role in modulating activities of PPAR-α and PPAR-γ These properties suggest that PPAR-*δ* agonists and antagonists may also have beneficial effects.

Studies have shown that PPAR ligands, particularly those that activate the alpha and gamma receptors (e.g., glitazone, troglitazone, pioglitazone, rosiglitazone, LY171883, 15d-prostaglandin J2, prostaglandin J2, bezafibrate and WY-14643), affect processes involved in restenosis. For example, thiazolidinediones inhibited vascular smooth muscle cell activation by angiotensin II by blocking angiotensin II-induced DNA synthesis. Y. Hattori et al., Biochem. Biophys. Res. Commun., 273: 1144-1149 (2000). Further, PPAR γ activators inhibited gene expression and migration in human vascular smooth muscle cells by decreasing MMP-9 expression and activity. N. Marx et al., Circ., Res., 83: 1097-1103 (1998). PPAR ligands also increased VEGF gene expression (K. Yamakawa, Biochem. Biophys. Res. Commun., 271: 571-575 (2000)) and blocked the G₁-S transition by inhibiting retinoblastoma protein phosphorylation (S. Wakino, J. Biol. Chem., 275: 22435-22441 (2000)). In addition, the PPAR-α agonist, GW-7647, inhibited atherosclerosis and formation of microphage-forming cells through liver X receptors (LXR). A. Li et al., J. Clin. Invest., 114: 1564-1576 (2004).

The present invention provides for the use of PPAR ligands to treat or prevent vascular disorders and related disorders. Such disorders include, but are not limited to, atherosclerosis, thrombosis, restenosis, hemorrhage, vascular dissection, vascular perforation, vascular aneurysm, vulnerable plaque, chronic total occlusion, patent foramen ovale, claudication, anastomotic proliferation for vein and artificial grafts, arteriovenous anastamoses, bile duct obstruction, ureter obstruction and tumor obstruction.

As an example, the anti-restenosis effect of PPAR ligands is likely due to the PPARs' multiple effects on differentiation, activation, migration and proliferation of restenosis-related cells, as well as the PPARs' effects on anti-inflammatory activities and lipid metabolism. The PPAR ligands may be PPAR agonists, antagonists, activators and/or repressors. The PPAR ligands can be used either in the general population of patients or in a population of patients with a high-risk of a particular disorder (e.g., restenosis among diabetic patients).

The invention covers a broad range of substances that act as PPAR ligands. Such ligands include full and partial agonists, antagonists, activators and repressors of any PPAR subtypes. The ligands are not necessarily structurally related; rather, they can include many families of chemicals. Non-limiting examples of PPAR ligands include rosiglitazone, pioglitazone, the thiazolidinedione (TZD) family of compounds, and non-TZD compounds.

Table 1 lists a number of synthetic ligands, fatty acids, eicosanoids and other substances that can activate PPARs. The present invention encompasses the use of each of these PPAR activators (i.e., agonists) to treat or prevent vascular disorders and related disorders.

**Table 1. Activators of PPARs**

| Class | Ligand | PPAR subtypes activated |
|---|---|---|
| Synthetic | WY-14643 | α, δ, γ |
| | GW-7647 | α |
| | clofibrates | α, δ, γ |
| | troglitazone | γ |
| | pioglitazone | γ |
| | rosiglitazone | δ, γ |
| Fatty acids | ETYA | α |
| | docahexanoic acid | α, δ, γ |
| | linoleic acid | α, δ |
| | arachidonic acid | α, δ, γ |
| Saturated fatty acids | C₆-C₁₈ | α, δ |
| Saturated fatty alcohols | C₁₄-C₁₆ | α |
| Oxidized linoleic acid products | 9-(R/S) HODE | γ |
| | 13-(R/S) HODE | γ |
| | 13-(S) HpODE | γ |
| | 9-oxo ODE | γ |
| | 13-oxo ODE | γ |
| Fatty acid CoA synthetase inhibitors | Triacsin C | α |
| Fatty acid CoA dehydrogenase inhibitors | nonythioacetic acid | α |
| | tetradecythioacetate | α |
| Carnitine palmitoyltransferase-1 inhibitors | 2-bromopalmitate | α, δ |
| | tetradecylglycidic acid | α, δ |
| Eicosanoids | PGJ2 | α, δ, γ |
| | 15d-PGJ2 | α, δ, γ |
| | prostacyclin (PGI₂) | α, δ, γ |
| | PGA_{1,2} | α, δ, γ |
| | PGB₂ | α, δ, γ |
| | 8-HEPE | α |
| | 8-(R) HETE | α, γ |
| | 8-(S) HETE | α, γ |
| | 12-HETE | α |
| | 15-HETE | γ |
| | LTB4 | α |
| Oxidized LDL | | γ |
| Others | NSAIDs | α, γ |

| | | |
|---|---|---|
| Abbreviations: ETYA = eicosatetraynoic acid HODE = hydroxyoctadecadienoic acid HpODE = hydroperoxyoctadecadienoic acid oxo ODE = oxidized octadecadienoic acid PG = prostaglandin 15d-PGJ2 = 15-deoxy-Δ^{12,14}-PGJ2 HEPE = hydroxyeicosapentaenoic acid HETE = hydroxyeicosatetaenoic acid LDL = low density lipoprotein NSAID = nonsteroidal anti-inflammatory drug | | |

PPAR ligands that can be used according to the present invention include thiazolidinethiones, glitazone, troglitazone, pioglitazone, rosiglitazone, and GW-7647.

The present invention encompasses various applications of PPAR ligands-e.g., the use of a single PPAR ligand, or the use of any combination of agonists and/or antagonists of one, two or all three PPAR subtypes. For example, the invention encompasses the use of a single ligand of any PPAR subtype, or a combination of ligands of one PPAR subtype, two PPAR subtypes or all three PPAR subtypes. The single PPAR ligand and multiple PPAR ligands can be agonists and/or antagonists of any PPAR subtypes.

For example, the PPAR ligand(s) can be:
- at least one PPAR-α agonist or antagonist;
- at least one PPAR-*β*/*δ* agonist or antagonist;
- at least one PPAR-γ agonist or antagonist;
- a combination of PPAR-α and PPAR-γ ligands - agonists and/or antagonists;
- a combination of PPAR-α and PPAR-β/δ ligands - agonists and/or antagonists;
- a combination of PPAR-γ and PPAR-β/δ ligands - agonists and/or antagonists; or
- a combination of PPAR-α, PPAR-*β*/*δ* and PPAR-γ. ligands - agonists and/or antagonists.

One expected benefit of the combination approach is that ligand(s) of one PPAR subtype can complement ligand(s) of other PPAR subtype(s). Non-limiting examples of combinations of PPAR ligands follow:
- a combination of at least one PPAR-α agonist or antagonist and at least one PPAR-γ agonist or antagonist;
- a combination of at least one PPAR-α agonist or antagonist and at least one PPAR-β/δ agonist or antagonist;
- a combination of at least one PPAR-γ agonist or antagonist and at least one PPAR- β/δ agonist or antagonist; and
- a combination of at least one PPAR-*α* agonist or antagonist, at least one PPAR-γ. agonist or antagonist, and at least one PPAR-β/δ agonist or antagonist.

The present invention encompasses both systemic and local administration of PPAR ligands. In one embodiment, the ligands are administered systemically. In another embodiment, the ligands are administered locally. More localized effects may be obtained by delivering the ligands to the diseased site locally using implantable devices (e.g., a local delivery catheter or a drug-delivery stent formed of a material (e.g., a polymeric coating) containing the ligands).

In a further embodiment, PPAR expression vectors that introduce into target cells the gene(s) for one or more PPAR ligands are delivered to a target area (e.g., to the site where a balloon or stent will be placed) and allowed to be expressed locally. The PPAR expression vectors can be delivered to the target site by any of various means known in the art, e.g., naked DNA, lipoplexes, micelles, particles, vesicles, or viral vectors (e.g., retroviruses, adenoviruses and adeno-associated viruses). In some embodiments, an implantable device containing at least one PPAR ligand (e.g., a drug-delivery stent containing at least one PPAR ligand impregnated in a polymeric coating on the surface of the stent) is implanted in a patient, or PPAR expression vectors are delivered to the target area. In other embodiments, both a PPAR ligand-containing device is implanted and PPAR expression vectors are delivered to the target area (e.g., the site where the PPAR ligand-coated stent will be implanted). The PPAR expression vectors are expected to further induce the beneficial effects of PPARs at the site of device implantation.

### Synthetic and Natural Retinoids

Another class of nuclear receptor agonists is retinoids that activate retinoic acid receptors. Natural retinoids, especially retinoic acid, have been used experimentally for the treatment of solid tumors, and cell culture experiments have shown their growth-inhibiting effects on smooth muscle cells. Natural retinoids may be relatively unstable, however, and may cause some local irritation, which may make them less suitable for local delivery from an implantable device (e.g., a stent).

Synthetic retinoids possessing increased stability and reduced irritation potential compared to natural retinoids have been developed and approved for human use. Endowed with such properties, synthetic retinoids may be suitable candidates for delivery from an implantable device. An additional advantage of synthetic retinoids is receptor specificity.

A synthetic retinoid possessing such advantageous features is tazarotene. For example, tazarotene is specific for retinoic acid receptors (RARs). RAR reactivity has been linked to the inhibition of smooth muscle cells, but not endothelial cells, in studies with other retinoids.

Studies have demonstrated that natural and synthetic retinoids can inhibit migration, proliferation and extracellular matrix deposition of smooth muscle cells. For instance, all-*trans* retinoic acid inhibited vascular smooth muscle cell proliferation. J. Pakala, J. Cardiovasc. Pharm., 35: 302 (2000). Other studies have shown that treatment of balloon-dilated rabbit femoral arteries with retinol resulted in larger internal and external elastic lamina 28 days after angioplasty. Wiegman et al., Arterioscl. Thromb. Vasc. Biol., 20: 89-95 (2000). In another study, *all-trans* retinoic acid and 13*-cis* retinoic acid suppressed intimal thickening and increased vessel remodeling in balloon-denuded rat carotid arteries. DeRose, Cardiovasc. Surg., 7: 633-639 (1999). A further study showed that all trans-retinoic acid reduced neointimal formation and promoted favorable geometric remodeling after balloon injury to rat carotid arteries. Miano et al., Circulation, 98a: 1219-1227 (1998).

The present invention provides for the use of synthetic and natural retinoids for the treatment or prevention of vascular disorders and related disorders. Such disorders include, but are not limited to, atherosclerosis, thrombosis, restenosis, hemorrhage, vascular dissection, vascular perforation, vascular aneurysm, vulnerable plaque, chronic total occlusion, patent foramen ovale, claudication, anastomotic proliferation for vein and artificial grafts, arteriovenous anastamoses, bile duct obstruction, ureter obstruction and tumor obstruction.

The retinoids can be used for local treatment of any of the vascular disorders described herein (e.g., restenosis) at all affected vascular sites, and after all applicable intravascular procedures.

The present invention encompasses both systemic and local administration of the retinoids. In one embodiment, the retinoids are administered systemically. In another embodiment, the retinoids are administered locally. More localized effects may be obtained by delivering the retinoids to the diseased site locally using implantable devices (e.g., a local delivery catheter or a drug-delivery stent formed of a material (e.g., a polymeric coating) containing the retinoids).

Individual retinoids can be used to obtain a beneficial therapeutic or prophylactic effect. However, a more beneficial effect, i.e., an additive or synergistic effect, may be obtained by using multiple retinoids. Retinoids that activate retinoic acid receptors are nuclear receptor agonists, and the synergistic action of a combination of these agonists can be expected. Tazarotene is an example of a retinoid that can be used alone or in combination with one or more other retinoids (e.g., glucocorticoids or glitazones) for the treatment or prevention of disorders described herein.

The structure of the synthetic retinoid, tazarotene, is provided below: 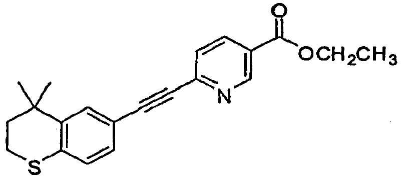 A summary of the properties of tazarotene is provided in Table 2 below. The properties of tazarotene indicate that it is a potentially good candidate for delivery from an implantable device (e.g., a stent coated with a polymeric material impregnated with tazarotene). Tazarotene is rapidly metabolized to tazarotenic acid in the blood, which may lead to a short local half-life.

**Table 2. Pharmacological properties of tazarotene**

| | |
|---|---|
| Primary indication | Anti-psoriasis, anti-acne |
| Primary mode of action | Anti-proliferative retinoid |
| Potency | Probably reasonably high anti-proliferative effect on fibroblasts at 1 micromolar |
| Pharmacokinetics | Unclear. Rapid de-esterification occurs in blood, but may not happen in tissue |

The discussion above on nuclear receptor ligands, PPAR ligands and retinoids provides a framework for various embodiments of the present invention, as described in further detail below.

### Implantable Device

Some embodiments of the invention, optionally in combination with one or more other embodiments described herein, are directed to an implantable device formed of a material comprising at least one biologically active agent selected from nuclear receptor ligands, nuclear receptor agonists and nuclear receptor antagonists that affect local gene expression or transcription at the site of implantation of the device. The ligands may be full or partial agonists or antagonists and may be activators or repressors of the particular nuclear receptors. In an embodiment, the least one biologically active agent includes at least one nuclear receptor agonist. In another embodiment, the least one biologically active agent includes at least one nuclear receptor antagonist.

In some embodiments, optionally in combination with one or more other embodiments described herein, the at least one biologically active agent includes at least one nuclear receptor ligand, nuclear receptor agonist or nuclear receptor antagonist that affects gene expression or transcription associated with a vascular condition or disorder or a related condition or disorder. In an embodiment, such conditions or disorders are selected from atherosclerosis, thrombosis, restenosis, hemorrhage, vascular dissection, vascular perforation, vascular aneurysm, vulnerable plaque, chronic total occlusion, patent foramen ovale, claudication, anastomotic proliferation for vein and artificial grafts, arteriovenous anastamoses, bile duct obstruction, ureter obstruction and tumor obstruction. In a specific embodiment, the condition or disorder is atherosclerosis, thrombosis, restenosis or vulnerable plaque.

In certain embodiments, optionally in combination with one or more other embodiments described herein, the material (e.g., a coating) forming the implantable device, or the device itself, does not comprise an antiproliferative agent and an anti-inflammatory agent, at least one of which is a nuclear receptor ligand, nuclear receptor agonist or nuclear receptor antagonist. In a particular embodiment, the antiproliferative agent is rapamycin, everolimus or a derivative thereof, and the anti-inflammatory agent is estradiol, 17-β estradiol, idoxifene, dexamethasone or clobetasol.

In other embodiments, optionally in combination with one or more other embodiments described herein, the at least one biologically active agent is a combination of biologically active agents selected from nuclear receptor ligands, nuclear receptor agonists and nuclear receptor antagonists. In one embodiment, the combination of biologically active agents is a combination of at least two nuclear receptor agonists. In another embodiment, the combination of biologically active agents is a combination of at least two nuclear receptor antagonists. In yet another embodiment, the combination of biologically active agents is a combination of at least one nuclear receptor agonist and at least one nuclear receptor antagonist.

In a further embodiment, optionally in combination with one or more other embodiments described herein, the combination of biologically active agents that are nuclear receptor ligands is not a combination of an antiproliferative agent and an anti-inflammatory agent. In still another embodiment, optionally in combination with one or more other embodiments described herein, the combination of biologically active agents is not a combination of an antiproliferative agent and estradiol or clobetasol.

In certain embodiments, optionally in combination with one or more other embodiments described herein, the at least one biologically active agent includes at least one nuclear receptor ligand selected from the group consisting of thiazolidinethiones, glitazone, troglitazone, pioglitazone, rosiglitazone. In other embodiments, the at least one bioactive agent includes at least one nuclear receptor ligand for the estrogen receptor, retinoic acid receptor, corticosteroid receptor, thyroid hormone receptor, PPAR, androgen receptor or vitamin D receptor that is not described herein. In yet other embodiments, the at least one bioactive agent includes at least one nuclear receptor ligand for other nuclear receptors associated with vascular disorders or related disorders that are not described herein.

In some embodiments, optionally in combination with one or more other embodiments described herein, the at least one biologically active agent specifically cannot be one or more of any of the nuclear receptor ligands, nuclear receptor agonists and nuclear receptor antagonists described herein. For example, in an embodiment, the at least one bioactive agent does not include estradiol, 17-β estradiol, idoxifene, dexamethasone or clobetasol.

In other embodiments, optionally in combination with one or more other embodiments described herein, the at least one biologically active agent includes at least one PPAR ligand, PPAR agonist or PPAR antagonist. In one embodiment, the at least one biologically active agent includes at least one PPAR agonist. In another embodiment, the at least one biologically active agent includes at least one PPAR antagonist. In yet another embodiment, the at least one biologically active agent is a combination of at least one PPAR agonist and at least one PPAR antagonist.

The at least one biologically active agent includes at least one PPAR agonist selected from synthetic PPAR agonists. In a particular embodiment, the synthetic PPAR agonists include troglitazone, pioglitazone and rosiglitazone.

In a particular embodiment, optionally in combination with one or more other embodiments described herein, the at least one biologically active agent includes at least one PPAR ligand, PPAR agonist or PPAR antagonist selected from the group consisting of thiazolidinethiones, glitazone, troglitazone, pioglitazone, rosiglitazone. ciprofibrate, gemfibrozil.

In some embodiments, optionally in combination with one or more other embodiments described herein, the at least one biologically active agent specifically cannot be one or more of any of the PPAR ligands, PPAR agonists and PPAR antagonists described herein.

In further embodiments, optionally in combination with one or more other embodiments described herein, the at least one biologically active agent includes at least one synthetic or natural retinoid. In one embodiment, the at least one biologically active agent includes at least one natural retinoid. In another embodiment, the at least one biologically active agent includes at least one synthetic retinoid. In a particular embodiment, the at least one synthetic retinoid includes tazarotene. In yet another embodiment, the at least one biologically active agent is a combination of at least one natural retinoid and at least one synthetic retinoid.

In some embodiments, the material (e.g., a coating) forming the implantable device, or the device itself, contains a combination of tazarotene and at least one additional retinoid. In one embodiment, the at least one additional retinoid includes at least one synthetic retinoid. In another embodiment, the at least one additional retinoid includes at least one natural retinoid. In a particular embodiment, the at least one additional retinoid is selected from the group consisting of 13-*cis* retinoic acid, all-*trans* retinoic acid, glucocorticoids and glitazones.

In certain embodiments, optionally in combination with one or more other embodiments described herein, the at least one biologically active agent specifically cannot be one or more of any of the synthetic retinoids and natural retinoids described herein.

In some embodiments, optionally in combination with one or more other embodiments described herein, the implantable device is for use in the treatment or prevention of a condition or disorder selected from atherosclerosis, thrombosis, restenosis, hemorrhage, vascular dissection, vascular perforation, vascular aneurysm, vulnerable plaque, chronic total occlusion, patent foramen ovale, claudication, anastomotic proliferation for vein and artificial grafts, arteriovenous anastamoses, bile duct obstruction, ureter obstruction and tumor obstruction. In a specific embodiment, the implantable device is for use in the treatment or prevention of atherosclerosis, thrombosis, restenosis or vulnerable plaque.

The implantable device formed of a material comprising at least one biologically active agent may be any device that can be implanted in an animal. Examples of implantable devices include, but are not limited to, stents (e.g., coronary stents and peripheral stents), grafts (e.g., aortic grafts, arterio-venous grafts and by-pass grafts), stent-grafts, catheters, leads and electrodes for pacemakers and defibrillators, endocardial leads (e.g., FINELINE and ENDOTAK, available from Abbott Vascular, Santa Clara, California), clips (e.g., anastomotic clips), shunts (e.g., cerebrospinal fluid and axius coronary shunts), closure devices (e.g., arterial and patent foramen ovale closure devices), valves (e.g., artificial heart valves), particles (e.g., bioactive-eluting particles), microparticles, nanoparticles, gels, and emulsions.

In an embodiment, optionally in combination with one or more other embodiments described herein, the implantable device is selected from stents, grafts, stent-grafts, catheters, leads and electrodes, clips, shunts, closure devices, valves, particles (e.g., bioactive-eluting particles), microparticles, nanoparticles, gels, and emulsions. In a more specific embodiment, optionally in combination with one or more other embodiments described herein, the implantable device is a stent. The stent may be balloon-expandable or self-expandable. Moreover, the stent may be intended for any vessel in the body, e.g., neurological, carotid, vein graft, synthetic graft, arteriovenous anastamosis, coronary, aortic renal, iliac, femoral, popliteal vasculature and urethral passages.

The underlying structure of the implantable device can be of virtually any design. The device can be made of a metallic material, an alloy, a polymeric material, any other type of material, or a combination thereof, as is known in the art. Moreover, the material comprising at least one biologically active agent may be used to make a portion of the implantable device or the whole device itself.

Non-limiting examples of metallic materials and alloys suitable for fabricating implantable devices include cobalt-chromium alloys (e.g., ELGILOY), "L-605", stainless steel (316L), "MP35N," "MP20N," ELASTINITE (Nitinol), tantalum, tantalum-based alloys, nickel-titanium alloys, platinum, platinum-based alloys (e.g., platinum-iridium alloy), iridium, gold, magnesium, titanium, titanium-based alloys, zirconium-based alloys, or combinations thereof. "L-605" is a trade name for an alloy of cobalt, chromium, tungsten, nickel and iron available as Haynes 25 from Haynes International (Kokomo, Indiana). "L-605" consists of 51 % cobalt, 20% chromium, 15% tungsten, 10% nickel and 3% iron. "MP35N" and "MP20N" are trade names for alloys of cobalt, nickel, chromium and molybdenum available from Standard Press Steel Co. (Jenkintown, Pennsylvania). "MP35N" consists of 35% cobalt, 35% nickel, 20% chromium and 10% molybdenum. "MP20N" consists of 50% cobalt, 20% nickel, 20% chromium and 10% molybdenum.

If a polymeric material is used to make a portion of the implantable device or the whole device itself, the polymeric material may comprise, e.g., a pure homopolymer, a copolymer, a blend of different types of polymers, a blend ofpolymer(s) and additional substance(s), or a combination thereof, as is known in the art. Further, additional polymer(s) and/or additional substance(s) may be attached to the underlying polymer forming the device or a portion thereof. The additional polymer(s) and/or additional substance(s) that may be attached to, grafted to, blended with, co-polymerized with, or incorporated with the underlying polymer may include, but are not limited to, biocompatible polymers, bioabsorbable polymers, biocompatible moieties, non-fouling moieties, and biobeneficial substances and materials. To enhance the mechanical characteristics (e.g., strength and rigidity) of an implantable device made substantially of a polymeric material, the device may be supported by additional structure(s) (e.g., struts in the case of stents made completely of a polymeric material).

There are various ways by which an implantable device may contain and deliver a bioactive agent. In one embodiment, optionally in combination with one or more other embodiments described herein, the at least one bioactive agent is attached to, blended with, impregnated in, or encapsulated in the polymeric material forming a portion of the implantable device or the whole device itself.

In another embodiment, optionally in combination with one or more other embodiments described herein, the at least one biologically active agent is embedded in micropores or nanopores on and/or in the implantable device. The micropores or nanopores can be created and/or be present on and/or in a device made of a metallic material, an alloy, a polymeric material, any other type of material, or a combination thereof.

### Coating Disposed over Implantable Device

The material comprising at least one biologically active agent may also be used to make a coating that is disposed over at least a portion of the implantable device. Accordingly, in an embodiment, optionally in combination with one or more other embodiments described herein, the material comprising at least one biologically active agent is a coating disposed over at least a portion of the implantable device.

The bioactive agent-containing material forming the coating may be a polymeric material comprising, e.g., a pure homopolymer, a copolymer, a blend of different types of polymers, a blend of polymer(s) and additional substance(s), or a combination thereof, as is known in the art. Further, additional polymer(s) and/or additional substance(s) may be attached to the underlying polymer of the coating. The additional polymer(s) and/or additional substance(s) that may be attached to, grafted to, blended with, co-polymerized with, or incorporated with the underlying polymer may include, but are not limited to, biocompatible polymers, bioabsorbable polymers, biocompatible moieties, non-fouling moieties, and biobeneficial substances and materials.

The at least one bioactive agent may be attached to, blended with, or impregnated in the polymeric material forming the coating. In an embodiment, optionally in combination with one or more other embodiments described herein, the bioactive agent-containing coating is disposed over an implantable device selected from stents, grafts, stent-grafts, catheters, leads and electrodes, clips, shunts, closure devices, and valves. In a particular embodiment, optionally in combination with one or more other embodiments described herein, the implantable device is a stent.

According to some embodiments of the invention, optionally in combination with one or more other embodiments described herein, a coating for an implantable device (e.g., a stent) may be a multi-layer structure that may include any of the following four layers or combination thereof:
(1) a primer layer;
(2) a drug-polymer layer (also referred to as a "reservoir" or "reservoir layer") or, alternatively, a polymer-free drug layer;
(3) a topcoat layer; and/or
(4) a finishing coat layer.

Each layer of a stent coating can be disposed over the stent by dissolving the polymer or a blend of polymers in a solvent, or a mixture of solvents, and disposing the resulting polymer solution over the stent by spraying or immersing the stent in the solution. After the solution has been disposed over the stent, the coating is dried by allowing the solvent to evaporate. The process of drying can be accelerated if the drying is conducted at an elevated temperature. The complete stent coating can be optionally annealed at a temperature between 40 °C and 150 °C for a period of time between 5 minutes and 60 minutes, if desired, to improve the thermodynamic stability of the coating.

To incorporate a bioactive agent (e.g., a drug) into the reservoir layer, the drug can be combined with the polymer solution that is disposed over the stent as described above. Alternatively, if it is desirable to have the stent coating with a fast drug-release rate, a polymer-free reservoir can be made. To fabricate a polymer-free reservoir, the drug can be dissolved in a suitable solvent or mixture of solvents, and the resulting drug solution can be disposed over the stent by spraying or immersing the stent in the drug-containing solution.

Instead of introducing a drug via a solution, the drug can be introduced as a colloid system, such as a suspension in an appropriate solvent phase. To make the suspension, the drug can be dispersed in the solvent phase using conventional techniques used in colloid chemistry. Depending on a variety of factors, e.g., the nature of the drug, those having ordinary skill in the art can select the solvent to form the solvent phase of the suspension, as well as the quantity of the drug to be dispersed in the solvent phase. Optionally, a surfactant may be added to stabilize the suspension. The suspension can be mixed with a polymer solution and the mixture can be disposed over the stent as described above. Alternatively, the drug suspension can be disposed over the stent without being mixed with the polymer solution.

The drug-polymer layer can be applied directly or indirectly over at least a part of the stent surface to serve as a reservoir for at least one bioactive agent (e.g., drug) that is incorporated into the reservoir layer. The optional primer layer can be applied between the stent and the reservoir to improve the adhesion of the drug-polymer layer to the stent. The optional topcoat layer can be applied over at least a portion of the reservoir layer and serves as a rate-limiting membrane that helps to control the rate of release of the drug. In one embodiment, the topcoat layer can be essentially free from any bioactive agents or drugs. If the topcoat layer is used, the optional finishing coat layer can be applied over at least a portion of the topcoat layer for further control of the drug-release rate and for improving the biocompatibility of the coating. Without the topcoat layer, the finishing coat layer can be deposited directly on the reservoir layer.

The process of the release of a drug from a coating having both topcoat and finishing coat layers includes at least three steps. First, the drug is absorbed by the polymer of the topcoat layer at the drug-polymer layer/topcoat layer interface. Next, the drug diffuses through the topcoat layer using the void volume between the macromolecules of the topcoat layer polymer as pathways for migration. Next, the drug arrives at the topcoat layer/finishing layer interface. Finally, the drug diffuses through the finishing coat layer in a similar fashion, arrives at the outer surface of the finishing coat layer, and desorbs from the outer surface. At this point, the drug is released into the blood vessel or surrounding tissue. Consequently, a combination of the topcoat and finishing coat layers, if used, can serve as a rate-limiting barrier. The drug can be released by virtue of the degradation, dissolution, and/or erosion of the layer(s) forming the coating, or via migration of the drug through non-degradable polymeric layer(s) into a blood vessel or tissue.

In one embodiment, any or all of the layers of the stent coating can be made of a biologically degradable/erodable/absorbable/resorbable polymer, a non-degradable/biostable polymer, or a combination thereof. In another embodiment, the outermost layer of the coating can be limited to a biodegradable polymer, a biostable polymer, or a combination thereof.

To illustrate in more detail, in a stent coating having all four layers described above (i.e., the primer, the reservoir layer, the topcoat layer and the finishing coat layer), the outermost layer is the finishing coat layer, which can be made of a biodegradable polymer, a biostable polymer, or a combination thereof. The remaining layers (i.e., the primer, the reservoir layer and the topcoat layer) optionally can also be fabricated of a biodegradable polymer, a biostable polymer, or a combination thereof. The polymer(s) in a particular layer may be the same as or different than those in any of the other layers.

If a finishing coat layer is not used, the topcoat layer can be the outermost layer and can be made of a biodegradable polymer, a biostable polymer, or a combination thereof. In this case, the remaining layers (i.e., the primer and the reservoir layer) optionally can also be fabricated of a biodegradable polymer, a biostable polymer, or a combination thereof. The polymer(s) in a particular layer may be the same as or different than those in any of the other layers.

If neither a finishing coat layer nor a topcoat layer is used, the stent coating could have only two layers - the primer and the reservoir. In such a case, the reservoir is the outermost layer of the stent coating and can be made of a biodegradable polymer, a biostable polymer, or a combination thereof. The primer optionally can also be fabricated of a biodegradable polymer, a biostable polymer, or a combination thereof. The two layers may be made from the same or different polymers.

Increased rate of degradation, erosion, absorption and/or resorption of a biologically degradable, erodable, absorbable and /or resorbable polymer is expected to lead to an increased rate of release of a drug due to the gradual disappearance of the polymer(s) that form the reservoir, the topcoat layer, and/or the finishing coat layer. Through appropriate selection of a biodegradable polymer, a biostable polymer or a combination thereof, a stent coating can be engineered to provide either fast or slow release of a drug, as desired. Those having ordinary skill in the art can determine whether a stent coating having slow or fast drug-release rate is advisable for a particular drug. For example, fast release may be recommended for stent coatings loaded with antimigratory drugs, which often need to be released within 1 to 2 weeks. For anti-proliferative and anti-inflammatory drugs, slower release may be desired, e.g., up to 30-day and 60-day release times, respectively.

Any layer of a stent coating may contain any amount of a bioabsorbable polymer and/or a biocompatible polymer, or a blend of more than one such polymer. Non-limiting examples of bioabsorbable polymers and biocompatible polymers include polyacrylates, e.g., poly(butyl methacrylate), poly(ethyl methacrylate), poly(ethyl methacrylate-co-butyl methacrylate), poly(acrylonitrile), poly(ethylene-co-methyl methacrylate), poly(acrylonitrile-co-styrene) and poly(cyanoacrylates); fluorinated polymers and/or copolymers, e.g., poly(vinylidene fluoride) and poly(vinylidene fluoride-co-hexafluoro propylene); poly(N-vinyl pyrrolidone); polydioxanone; polyorthoesters; polyanhydrides; poly(glycolic acid); poly(glycolic acid-co-trimethylene carbonate); polyphosphoesters; polyphosphoester urethanes; poly(amino acids); poly(trimethylene carbonate); poly(iminocarbonates); co-poly(ether-esters); polyalkylene oxalates; polyphosphazenes; biomolecules, e.g., fibrin, fibrinogen, cellulose, cellophane, starch, collagen, hyaluronic acid, and derivatives thereof (e.g., cellulose acetate, cellulose butyrate, cellulose acetate butyrate, cellulose nitrate, cellulose propionate, cellulose ethers, and carboxymethyl cellulose); polyurethanes; silicones; polyesters; polyolefins; polyisobutylene and ethylene-alphaolefin copolymers; vinyl halide polymers and copolymers, e.g., polyvinyl chloride; polyvinyl ethers, e.g., polyvinyl methyl ether; polyvinylidene chloride; polyvinyl ketones; polyvinyl aromatics, e.g., polystyrene; polyvinyl esters, e.g., polyvinyl acetate; copolymers of vinyl monomers with each other and olefins, e.g., poly(ethylene-co-vinyl alcohol) (EVAL); ABS resins; poly(ethylene-co-vinyl acetate); polyamides, e.g., Nylon 66 and polycaprolactam; alkyd resins; polycarbonates; polyoxymethylenes; polyimides; polyethers, epoxy resins; polyurethanes; rayon; and rayon-triacetate.

Any layer of a stent coating may also contain any amount of a non-degradable polymer, or a blend of more than one such polymer. Non-limiting examples of non-degradable polymers include methylmethacrylate, ethylmethacrylate, butylmethacrylate, 2-ethylhexylmethacrylate, laurylmethacrylate, hydroxyl ethyl methacrylate, polyethylene glycol (PEG) acrylate, PEG methacrylate, 2-methacryloyloxyethylphosphorylcholine (MPC) and n-vinyl pyrrolidone, methacrylic acid, acrylic acid, hydroxypropyl methacrylate, hydroxypropylmethacrylamide, 3-trimethylsilylpropyl methacrylate, and copolymers thereof.

In some embodiments, optionally in combination with one or more other embodiments described herein, the at least one biologically active agent is embedded in micropores or nanopores on and/or in the coating that is disposed over the implantable device. For example, an amorphous glass harboring micropores or nanopores in its structure may be used as a bioactive agent reservoir. The amorphous glass can be coated over an implantable device as described above. The device can then be immersed in a solution of the at least one bioactive agent and solvent for 1 hour. The solvent can then be evaporated in a vacuum oven or a convection oven at elevated temperature (e.g., 50 °C) for 1 hour. In certain embodiments, the amorphous glass reservoir layer can be coated with either a polymeric topcoat layer or a nanoporous amorphous glass topcoat layer, as described above.

### Bioactive-Release Profile

In some embodiments, optionally in combination with one or more other embodiments described herein, the implantable device of the invention can have any one or a combination of a pulse, burst and sustained release profile. For example, the device can be configured to have a pulse or burst release of a bioactive agent, followed by a sustained release of the same agent.

The term "pulse release" generally refers to a release profile of a bioactive agent that features a sudden surge in the release rate of the agent. The surge in the release rate of the agent would then disappear within a period of time. A more detailed definition of the term can be found in Encyclopedia of Controlled Drug Delivery, Edith Mathiowitz, Ed., Culinary and Hospitality Industry Publications Services, which is incorporated by reference in its entirety herein.

In certain embodiments, the term "fast release" refers to *in vivo* release of substantially the entire amount of a bioactive agent from an implantable device (e.g., from the coating disposed over the device) in 15 days or less, e.g., within 7 to 14 days. The term "fast release" is used interchangeably with the term "burst release".

The term "sustained release" generally refers to a release profile of a bioactive agent that can include zero-order release, exponential decay, step-function release or other release profiles that carry over a period of time, e.g., ranging from several days to several weeks or years. The terms "zero-order release", "exponential decay" and "step-function release" as well as other sustained release profiles are well known in the art. See, e.g., Encyclopedia of Controlled Drug Delivery, Edith Mathiowitz, Ed., Culinary and Hospitality Industry Publications Services.

In one embodiment, optionally in combination with one or more other embodiments described herein, the implantable device of the invention has a sustained release profile. In certain embodiments, the device provides sustained release of the at least one biologically active agent over a period up to 24 months, or up to 18 months, or up to 12 months, or up to 6 months, or up to 3 months, or up to 2 months, or up to 1 month.

The release rate of a bioactive agent can be tailored by various means. For example, the release rate of an agent can be tailored by the coating concentration of the agent and the equilibrium water uptake of the barrier if the barrier is formed of a hydrophobic, nonabsorable polymer or the absorption rate if the barrier is formed of an absorbable polymer.

In some embodiments where the implantable device is formed of a material comprising two or more bioactive agents, one of the agents or more than one of them can have any one or a combination of a pulse, burst or sustained release profile. The device (e.g., the coating disposed thereover) can have a release profile that features a burst release of one or more agents together with a sustained release of the one or more agents. In an embodiment, the device can be configured to have a profile of a burst release of a first agent and sustained release of the first agent and a second agent. In another embodiment, the device can be designed to have a burst release of two agents followed by a sustained release of both agents. In yet another embodiment, the device can be configured to provide a pulse release of one or more agents and optionally a sustained release of the same or different agents.

In further embodiments, optionally in combination with one or more other embodiments described herein, the implantable device (e.g., the coating disposed thereover) is capable of simultaneously releasing two or more bioactive agents. Simultaneous delivery means that there is at least some overlap in the release of the agents. Under this embodiment, one of the agents can be released first such as by pulse, burst, or sustained release so long as there is an overlap in release with the second agent.

A coating capable of simultaneously releasing two or more bioactive agents can have a variety of configurations. For example, the coating can have a layer that comprises a mixture of two agents, or it can have two layers, each of which comprises a particular bioactive agent and a polymer that may be the same as or different than the polymer in the other layer.

### Method of Treating or Preventing Disorders

An implantable device formed of a material comprising at least one nuclear receptor ligand that affects local gene expression or transcription at the site of device implantation can be used to treat, prevent or diagnose vascular conditions or disorders and related conditions or disorders. Examples of such conditions or disorders include, but are not limited to, atherosclerosis, thrombosis, restenosis, hemorrhage, vascular dissection, vascular perforation, vascular aneurysm, vulnerable plaque, chronic total occlusion, patent foramen ovale, claudication, anastomotic proliferation of vein and artificial grafts, arteriovenous anastamoses, bile duct obstruction, ureter obstruction and tumor obstruction.

Accordingly, some embodiments of the disclosure are directed to a method of treating or preventing a condition or disorder in a patient, comprising implanting in the patient an implantable device, wherein:
the implantable device is formed of a material comprising at least one biologically active agent selected from nuclear receptor ligands, nuclear receptor agonists and nuclear receptor antagonists that affect local gene expression or transcription at the site of implantation, and
the condition or disorder is selected from the group consisting of atherosclerosis, thrombosis, restenosis, hemorrhage, vascular dissection, vascular perforation, vascular aneurysm, vulnerable plaque, chronic total occlusion, patent foramen ovale, claudication, anastomotic proliferation for vein and artificial grafts, arteriovenous anastamoses, bile duct obstruction, ureter obstruction and tumor obstruction.

The whole discussion herein, including all of the various embodiments described herein, relating to the implantable device and the material (e.g., the coating) forming the device also apply to the inventive method of treatment or prevention. For example, in certain embodiments of the method, the material (e.g., a coating) forming the implantable device, or the device itself, does not comprise an antiproliferative agent and an anti-inflammatory agent, at least one of which is a nuclear receptor ligand, nuclear receptor agonist or nuclear receptor antagonist. In a particular embodiment of the method, the antiproliferative agent is rapamycin, everolimus or a derivative thereof, and the anti-inflammatory agent is estradiol, 17-β estradiol, idoxifene, dexamethasone or clobetasol.

As another example, in some embodiments of the method, the at least one biologically active agent specifically cannot be one or more of any of the nuclear receptor ligands, nuclear receptor agonists and nuclear receptor antagonists described herein. For example, in one embodiment, the at least one bioactive agent that is a nuclear receptor ligand does not include estradiol, 17-β estradiol, idoxifene, dexamethasone or clobetasol. In other embodiments of the method, the at least one biologically active agent specifically cannot be one or more of any of the PPAR ligands, PPAR agonists and PPAR antagonists described herein. In yet other embodiments of the method, the at least one biologically active agent specifically cannot be one or more of any of the synthetic retinoids and natural retinoids described herein.

As a further example, in an embodiment of the method, the combination of biologically active agents that are nuclear receptor ligands is not a combination of an antiproliferative agent and an anti-inflammatory agent. In another embodiment of the method, the combination of biologically active agents is not a combination of an antiproliferative agent and estradiol or clobetasol.

As yet another example, in one embodiment of the method, the implantable device has a bioactive agent-release profile that includes one or a combination of a pulse, burst and sustained release profile. In certain embodiments of the method, the implantable device provides sustained release of the at least one bioactive agent over a period up to 24 months, or up to 12 months, or up to 6 months, or up to 3 months, or up to 2 months, or up to 1 month. In a further embodiment of the method, the implantable device is capable of simultaneously releasing two or more bioactive agents.

As still another example, in an embodiment of the method, the at least one biologically active agent is embedded in micropores or nanopores on and/or in the implantable device. In another embodiment of the method, the material comprising the at least one bioactive agent is a coating disposed over at least a portion of the implantable device. In a specific embodiment of the method, the implantable device is a stent. In another specific embodiment of the method, the condition or disorder is atherosclerosis, thrombosis, restenosis or vulnerable plaque.

The dosage or concentration of the at least one bioactive agent required to produce a favorable therapeutic effect should be less than the level at which the bioactive agent produces toxic effects and greater than the level at which non-therapeutic results are obtained. The dosage or concentration of the bioactive agent required to, e.g., inhibit the target cellular activity of the vascular region can depend upon various factors such as the particular circumstances of the patient, the nature of the trauma, the nature of the therapy desired, the time over which the administered agent resides at the vascular site, and if other active agents are employed, the nature and type of the substance or combination of substances. Therapeutically effective concentrations or dosages can be determined empirically, e.g., by infusing vessels from suitable animal model systems and using immunohistochemical, fluorescent or electron microscopy methods to detect the agent and its effects, or by conducting suitable *in vitro* studies. Standard pharmacological test procedures to determine concentrations or dosages are understood by one of ordinary skill in the art.

The mode of delivery of a bioactive agent or a combination of bioactive agents can be local or systemic. Local administration can be by means of an implantable device (e.g., a bare metal stent, a coated stent, or a biodegradable/bioabsorbable stent), a drug-delivery particle or other known methods of local drug delivery. Systemic administration can be accomplished by various means known in the art, e.g., orally or parenterally, including intravascularly. For example, a first bioactive agent can be delivered by a stent and another bioactive agent by a catheter at the site of treatment. The delivery can be simultaneous or in sequence. For example, one bioactive agent can be delivered before another bioactive agent while there is some or a significant overlap between the deliveries of both agents. The drug treatment can be by means of an implantable device (e.g., a stent).

### Use of Nuclear Receptor Ligands in the Manufacture of Implantable Devices

At least one nuclear receptor ligand that affects gene expression or transcription can be used in the manufacture of an implantable device that is useful in the treatment, prevention or diagnosis of vascular disorders and related disorders. A material comprising at least one nuclear receptor ligand that affects gene expression or transcription can also be used in the manufacture of a portion (e.g., a coating) of an implantable device, or the whole device, which has such utility.

Accordingly, further embodiments of the invention, optionally in combination with one or more other embodiments described herein, are directed to the use of at least one biologically active agent selected from nuclear receptor ligands, nuclear receptor agonists and nuclear receptor antagonists in the manufacture of an implantable device, wherein:
the nuclear receptor ligands, agonists and antagonists affect local gene expression or transcription at the site of device implantation, and
the device is for use in the treatment or prevention of a condition or disorder selected from atherosclerosis, thrombosis, restenosis, hemorrhage, vascular dissection, vascular perforation, vascular aneurysm, vulnerable plaque, chronic total occlusion, patent foramen ovale, claudication, anastomotic proliferation for vein and artificial grafts, arteriovenous anastamoses, bile duct obstruction, ureter obstruction and tumor obstruction.

Other embodiments of the invention, optionally in combination with one or more other embodiments described herein, are drawn to the use of a material comprising at least one nuclear receptor ligand that affects gene expression or transcription in the manufacture of a portion (e.g., a coating) of an implantable device, or the whole device, for use in the treatment or prevention of vascular disorders and related disorders.

The whole discussion herein, including all of the various embodiments described herein, relating to the implantable device, the material (e.g., the coating) forming the device, and the method of treating or preventing vascular and related disorders utilizing the device also apply to the use of at least one nuclear receptor ligand, or a material comprising at least one nuclear receptor ligand, in the manufacture of an implantable device for use in treating or preventing vascular and related disorders.

For example, in certain embodiments of the inventive use, the implantable device, or the material forming the device, does not comprise an antiproliferative agent and an anti-inflammatory agent, at least one of which is a nuclear receptor ligand, nuclear receptor agonist or nuclear receptor antagonist. In an embodiment, the antiproliferative agent is rapamycin, everolimus or a derivative thereof, and the anti-inflammatory agent is estradiol, 17-β estradiol, idoxifene, dexamethasone or clobetasol.

In some embodiments of the inventive use, the implantable device has a bioactive agent-release profile that includes one or a combination of a pulse, burst and sustained release profile for each of the at least one bioactive agent. In one embodiment, the device provides sustained release of each of the at least one bioactive agent. In certain embodiments, the device provides sustained release of the at least one bioactive agent over a period up to 24 months, or up to 12 months, or up to 6 months, or up to 3 months, or up to 2 months, or up to 1 month.

In other embodiments of the inventive use, the at least one bioactive agent is a combination of biologically active agents selected from nuclear receptor ligands, nuclear receptor agonists and nuclear receptor antagonists that affect local gene expression or transcription at the site of device implantation. In one embodiment, the combination of bioactive agents is a combination of at least one nuclear receptor agonist and at least one nuclear receptor antagonist. In another embodiment, the implantable device is capable of simultaneously releasing two or more biologically active agents.

In other embodiments of the inventive use, the at least one bioactive agent includes at least one PPAR ligand, PPAR agonist or PPAR antagonist. In an embodiment, the at least one PPAR ligand, PPAR agonist or PPAR antagonist is selected from thiazolidinethiones, glitazone, troglitazone, pioglitazone, rosiglitazone.

In yet other embodiments of the inventive use, the at least one bioactive agent includes at least one synthetic or natural retinoid. In one embodiment, the at least one synthetic or natural retinoid includes tazarotene. In another embodiment, the implantable device, or the material forming the device, comprises at least one other retinoid in addition to tazarotene. In an embodiment, the at least one additional retinoid is selected from 13-*cis* retinoic acid, all*-trans* retinoic acid, glucocorticoids and glitazones.

In some embodiments of the inventive use, the implantable device is selected from stents, grafts, stent-grafts, catheters, leads and electrodes, clips, shunts, closure devices, valves, particles, microparticles, nanoparticles, gels, and emulsions. In a particular embodiment, the device is a stent.

In certain embodiments of the inventive use, the at least one bioactive agent is embedded in micropores or nanopores on and/or in a portion of the implantable device or the whole device. In one embodiment, the micropores or nanopores are present in a metallic material or an alloy forming the device. In another embodiment, the micropores or nanopores are present in a polymeric material forming the device.

In other embodiments of the inventive use, the material comprising the at least one bioactive agent is a coating disposed over at least a portion of the implantable device. In a particular embodiment, the coated implantable device is a coated stent.

In a specific embodiment of the inventive use, the implantable device is for use in the treatment or prevention of atherosclerosis, thrombosis, restenosis or vulnerable plaque.

### Examples

The examples set forth below are shown for the sole purpose of further illustrating embodiments of the present invention and are in no way meant to limit the invention. The following examples are given to aid in understanding the invention, but it is to be understood that the invention is not limited to the particular materials or procedures of the examples.

### Example 1

A polymer solution containing between about 0.1 mass % and about 15 mass % (e.g., about 2.0 mass %) of a copolymer of ethylene and vinyl alcohol (EVAL) and the balance, N,N-dimethylacetamide (DMAC) solvent, can be prepared. The solution can be applied onto a stent to form a primer layer. To apply the primer layer, a spray apparatus, such as an EFD 780S spray nozzle with a VALVEMATE 7040 control system (manufactured by EFD, Inc. of East Providence, Rhode Island) can be used. The EFD 780S spray nozzle is an air-assisted external mixing atomizer. The composition is atomized by air and applied to the stent surfaces. During the process of applying the composition, the stent can be optionally rotated about its longitudinal axis, at a speed of 50 to about 150 rpm. The stent can also be linearly moved along the same axis during the application.

The EVAL solution can be applied to a 13-mm TETRA stent (available from Abbott Vascular Corp.) in a series of 10-second passes, to deposit, e.g., 10 µg of coating per spray pass. Instead of the 13-mm TETRA stent, another suitable stent can be used, e.g., a 12-mm VISION stent (also available from Abbott Vascular Corp.). Between the spray passes, the stent can be dried for about 10 seconds using flowing air with a temperature of about 60 °C. Five spray passes can be applied, followed by baking the primer layer at about 140 °C for one hour. As a result, a primer layer can be formed having a solids content of about 50 µg. "Solids" means the amount of the dry residue deposited on the stent after all volatile organic compounds (e.g., the solvent) have been removed.

A drug-containing formulation can be prepared comprising:
(a) between 0.1 mass % and 15 mass % (e.g., about 2.0 mass %) of EVAL;
(b) between 0.1 mass % and 2 mass % (e.g., about 1.0 mass %) of a bioactive agent; and
(c) the balance, a solvent mixture of DMAC and pentane, the solvent mixture containing about 80 mass % of DMAC and about 20 mass % of pentane.

In a manner identical to the application of the primer layer, five spray passes can be performed, followed by baking the drug-polymer layer at about 50 °C for about 2 hours, to form the drug-polymer layer having a solids content between 30 µg and 750 µg (e.g., about 90 µg) and a drug content of between 10 µg and 250 µg (e.g., about 30 µg).

Finally, a topcoat composition to control the drug-release rate can be prepared, comprising between 0.1 mass % and 15 mass % (e.g., about 2.0 mass %) of poly(n-butyl methacrylate) (PBMA) and the balance a solvent system, e.g., a solvent system including a 10:50:40 (mass) blend of acetone, Techspray's FLUX REMOVER AMS, and xylene. In a manner identical to the application of the primer layer and the drug-polymer layer, a number of spray passes are performed followed by final baking at about 50 °C for about 2 hours. As a result, the topcoat membrane can be formed, the membrane having a solids content of between 30 µg and 350 µg (e.g., about 50 µg).

### Example 2

A stent was coated as described in Example 1, wherein estradiol was used. The coated stent was studied for drug release. The stent was immersed for 24 hours in bovine serum. Estradiol was extracted, and the amount of estradiol released after 24 hours was measured by HPLC. The results of this study are summarized in Table 3 below.

**Table 3. Drug-release study of stent coatings having PBMA topcoat membranes (EVAL-based drug-polymer layer, estradiol drug)**

| No. | Topcoat Membrane Solids (µg) | Drug Loaded in the Drug-Polymer Layer (µg) | Percent of the Drug Released in 24 Hours |
|---|---|---|---|
| 1 | 30 | 240 | 15.0 |
| 2 | 50 | 240 | 13.0 |
| 3 | 100 | 240 | 11.0 |
| 4 | 160 | 240 | 4.3 |
| 5 | 300 | 170 | 1.5 |

In addition, a kinetic study of the drug release profile was conducted. The stent had a total amount of solids of the topcoat membrane of about 160 µg and a total amount of estradiol in the drug-polymer layer of about 30 µg. The stent was immersed in a phosphate-buffered saline solution having 1 mass % of sodium dodecyl sulfate. A sample of the solution was taken every 20 minutes and analyzed by HPLC for the amount of estradiol released.

Three different coated stents had reproducible drug-release profiles. After 10 days, about 50 mass % of estradiol was released in an almost perfectly linear profile, indicating a topcoat layer-controlled zero-order type of release. Specifically, about 13 mass % of estradiol was released after 2 days, 21% after 4 days, 29% after 6 days, 35% after 8 days, and 46% after 10 days. The small burst in the first 24 hours is due to the saturation of the topcoat layer with estradiol. Once a stable state was established, the release rate remained constant for 240 hours. The linear correlation coefficient between 24 and 240 hours was 0.997.

### Example 3

A drug-polymer layer comprising 17-β estradiol was applied onto a stent in the following manner. A first composition was prepared by mixing the following components:
(a) about 2.0 mass % of EVAL; and
(b) the balance, a mixture of solvents, DMAC and ethanol, in a ratio of DMAC to ethanol of about 70:30 by mass.

The first composition was applied onto the surface of a bare 13 mm TETRA stent (available from Abbott Vascular Corp.) by spraying and dried to form a primer layer. A spray coater having an EFD 7803 spray valve with 0.014 inch fan nozzle with a VALVEMATE 7040 control system (manufactured by EFD Inc. of East Providence, Rhode Island) was used. The fan nozzle was maintained at about 60 °C with a feed pressure of about 0.2 atm (about 3 psi) and an atomization pressure of about 1.35 atm (about 20 psi). An average of about 19 micrograms (*µ*g) per coating pass was applied and an average total of about 62 *µ*g of the wet coating was applied.

The primer layer was baked at about 140 °C for about one hour, yielding a layer with an average total amount of solids of about 61 *µ*g, corresponding to an average thickness on the stent of 0.65 *µ*m. "Solids" means the amount of dry residue deposited on the stent after all volatile organic compounds (e.g., the solvent) have been removed.

A second composition was prepared by mixing the following components:
(a) about 2.0 mass % of EVAL;
(b) about 0.7 mass % 17-β estradiol; and
(c) the balance, a mixture of solvents, DMAC and ethanol, in a ratio of DMAC to ethanol of about 70:30 by mass.

The second composition was applied onto the dried primer layer to form a drug-polymer layer using the same spraying technique and equipment used for the primer layer. About 497 *µ*g of the wet coating was applied, followed by drying at about 50 °C for about 2 hours. The total amount of solids of the drug-polymer layer was about 494 *µ*g, corresponding to an average thickness on the stent of about 5.3 *µ*m.

A third, topcoat composition was prepared by mixing the following components:
(a) about 1.2 g of a 10 mass % solution of KYNAR-FLEX 2800 in acetone;
(b) about 1.89 g of additional acetone;
(c) about 5.94 g of cyclohexanone; and
(d) about 2.97 g of AMS FLUX REMOVER.

The topcoat composition was applied by spraying using an EFD 7803 spray valve with 0.014 inch fan nozzle to form a topcoat layer, followed by drying. The nozzle temperature was at ambient temperature with a feed pressure of about 0.2 atm (3 psi) and an atomization pressure of about 1 atm (15 psi). The dryer temperature was at ambient temperature with a dryer air pressure of about 2.7 atm (40 psi). An average of about 5 *µ*g per coating pass was applied and an average total of about 60 *µ*g of wet coating was applied. The topcoat layer was baked at about 50 °C for two hours, yielding a total amount of solids of about 55 *µ*g, corresponding to a thickness of about 0.38 *µ*m.

Three stents coated according to this example were tested for their drug-release rate. The stents were immersed in individual, stirred vessels containing a phosphate-buffered saline solution that included about 1 mass % of sodium dodecyl sulfate. The buffer solution had a pH of about 7.4 thermostatted at 37 °C. The amount of 17-β estradiol released was determined at measured intervals of time by HPLC. The three stents displayed similar drug-release rates. There was an initial small burst of 17-β estradiol during the first 20 hours, after which the release rate became approximately linear. Specifically, about 18% of 17-β estradiol was released after 10 hr, 25% after 20 hr, 30% after 30 hr, 35% after 40 hr, 40% after 50 hr, 43% after 60 hr, and 45% after 70 hr.

## Claims

1. An implantable device comprising at least one biologically active agent selected from the group of PPAR ligands, PPAR agonists and PPAR antagonists for use in the treatment or prevention of a condition or disorder selected from the group consisting of atherosclerosis, thrombosis, restenosis, hemorrhage, vascular dissection, vascular perforation, vascular aneurysm, vulnerable plaque, chronic total occlusion, patent foramen ovale, claudication, anastomic proliferation for vein and artificial grafts, arteriovenous anastamoses, bile duct obstruction, ureter obstruction and tumor obstruction, wherein the biologically active agent affect local gene expression or transcription at the site of device implantation, and wherein the PPAR ligands, PPAR agonists and/or PPAR antagonists are selected from the group consisting of troglitazone, pioglitazone, and rosiglitazone.

2. The implantable device of claim 1, wherein the condition or disorder is selected from the group consisting of atherosclerosis, thrombosis, restenosis and vulnerable plaque.

3. The implantable device of any of the claims 1-2, wherein the biologically active agent is embedded in micropores or nanopores on and/or in the implantable device.

4. The implantable device of claim 1, wherein the release of the biologically active agent from the implantable device has a release profile that is one of or a combination of pulse, burst and sustained release.

5. The implantable device of claim 4, wherein the sustained release profile is selected from the group consisting of release for up to 3 months, up to 6 months or up to 24 months.

6. The implantable device of any of the claims 1-5, wherein the implantable device is selected from the group a stent, a graft, a stent-graft, a catheter, a lead and electrode, a clip, a shunt, a closure device, a valve, a particle, a microparticle, a nanoparticle, a gel and an emulsion.

7. The implantable device of claim 6, wherein the implantable device is a stent.

8. An implantable device comprising at least one biologically active agent selected from the group consisting of PPAR ligands, PPAR agonists and PPAR antagonists, wherein the device is a stent, a graft, a stent-graft, a catheter, a lead and electrode, a clip, a shunt, a closure device, a valve, a particle, a microparticle, a nanoparticle, a gel and an emulsion, and wherein the PPAR ligand, PPAR agonists and/or PPAR antagonist is selected from the group consisting of troglitazone, pioglitazone, and rosiglitazone.

9. The implantable device of claim 8, wherein the device is a stent.

## Patentansprüche

1. Implantierbare Vorrichtung umfassend mindestens einen Wirkstoff, der ausgewählt ist aus der Gruppe bestehend aus PPAR-Liganden, PPAR-Agonisten und PPAR-Antagonisten zur Verwendung bei der Behandlung oder Vorbeugung eines Zustands oder einer Störung, der/die ausgewählt ist aus der Gruppe bestehend aus Atherosklerose, Thrombose, Restenose, Hämorrhagien, Gefäßdissektion, Gefäßperforation, Gefäßaneurysma, vulnerablen Plaques, chronischem Totalverschluss, offenem Foramen ovale, Klaudication, anastomotischer Proliferation eines Gefäßes und künstlichen Implantaten, arteriovenösen Anastamosen, Gallengänge-Obstruktion, Harnröhrenobstruktion und tumorbedingten Obstruktion, wobei der Wirkstoff die lokale Genexpression oder Gentranskription an der Stelle der Vorrichtungsimplantation beeinflusst, und wobei die PPAR-Liganden, PPAR-Agonisten und/oder PPAR-Antagonisten ausgewählt aus der Gruppe bestehend aus Troglitazon, Pioglitazon, und Rosiglitazon sind.

2. Implantierbare Vorrichtung nach Anspruch 1, wobei der Zustand oder die Störung ausgewählt aus der Gruppe bestehend aus Atherosklerose, Thrombose, Restenose und vulnerablen Plaques ist.

3. Implantierbare Vorrichtung nach einem der Ansprüche 1-2, wobei der Wirkstoff in Mikroporen oder Nanoporen auf und/oder innerhalb der implantierbaren Vorrichtung eingebettet ist.

4. Implantierbare Vorrichtung nach Anspruch 1, wobei die Freisetzung des Wirkstoffes aus der implantierbaren Vorrichtung ein Freisetzungsprofil hat, das eins von Puls, Ausbruch und anhaltender Freisetzung oder eine Kombination davon ist

5. Implantierbare Vorrichtung nach Anspruch 4, wobei das Profil anhaltender Freisetzung ausgewählt aus der Gruppe bestehend aus Freisetzung in einem Zeitraum von bis 3 Monaten, bis 6 Monaten oder bis 24 Monaten ist.

6. Implantierbare Vorrichtung nach einem der Ansprüche 1-5, wobei die implantierbare Vorrichtung ausgewählt ist aus der Gruppe bestehend aus einem Stent, einem Implantat, einem Stent-Implantat, einem Katheter, einem Lead und Elektrode, einer Klemme, einem Shunt, einer Verschlussvorrichtung, einem Ventil, einer Partikel, einer Mikropartikel, einer Nanopartikel, einem Gel und einer Emulsion

7. Implantierbare Vorrichtung nach Anspruch 6, wobei die implantierbare Vorrichtung ein Stent ist.

8. Implantierbare Vorrichtung umfassend mindestens einen Wirkstoff, der ausgewählt ist aus der Gruppe bestehend aus PPAR-Liganden, PPAR-Agonisten und PPAR-Antagonisten, wobei die Vorrichtung ein Stent, ein Implantat, ein Stent-Implantat, ein Katheter, ein Lead und Elektrode, eine Klemme, ein Shunt, eine Verschlussvorrichtung, ein Ventil, eine Partikel, eine Mikropartikel, eine Nanopartikel, ein Gel und eine Emulsion ist, und wobei der PPAR-Ligand, die PPAR-Agonisten und/oder PPAR-Antagonisten ausgewählt aus der Gruppe bestehend aus Troglitazon, Pioglitazon, und Rosiglitazon sind.

9. Implantierbare Vorrichtung nach Anspruch 8, wobei die Vorrichtung ein Stent ist.

## Revendications

1. Dispositif implantable comprenant au moins un agent biologiquement actif choisi dans le groupe constitué des ligands des PPARs, des agonistes des PPARs et des antagonistes des PPARs pour l'utilisation dans le traitement ou prévention d'un état ou trouble choisi dans le groupe constitué de l'athérosclérose, la thrombose, la resténose, les hémorrhagies, la dissection vasculaire, la perforation vasculaire, l'anéurisme vasculaire, la plaque vulnérable, l'occlusion totale cronique, le foramen ovale perméable, la claudication, la prolifération anastomotique des veines et des greffes artificielles, les anastamoses artérioveineuses, l'obstruction du canal biliaire, l'obstruction des uretères et l'obstruction tumorale, dans lequel l'agent biologiquement actif affecte l'expression ou la transcription génétique locale à la position de l'implantation du dispositif, et dans lequel les ligands des PPARs, les agonistes des PPARs et/ou les antagonistes des PPARs sont choisis dans le groupe constitué de la troglitazone, la pioglitazone, et la rosiglitazone.

2. Le dispositif implantable de la revendication 1, dans lequel l'état ou le trouble est choisi parmi l'athérosclérose, la thrombose, la resténose et les plaques vulnérables.

3. Le dispositif implantable selon l'une quelconque des revendications 1-2, dans lequel l'agent biologiquement actif est incorporé dans des micropores ou nanopores sur et/ou dans le dispositif implantable.

4. Le dispositif implantable de la revendication 1, dans lequel la libération de l'agent biologiquement actif du dispositif implantable a un profil de libération qui est un ou une combinaison de libération pulsée, déchaînement et libération prolongée.

5. Le dispositif implantable de la revendication 4, dans lequel le profil de libération prolongée est choisi dans le groupe constitué de libération pendant jusqu'à 3 mois, jusqu'à 6 mois ou jusqu'à 24 mois.

6. Le dispositif implantable de l'une quelconque des revendications 1-5, dans lequel le dispositif implantable est choisi dans le groupe constitué d'une endoprothèse vasculaire, d'une greffe, d'une endoprothèse vasculaire-greffe, d'un cathéter, d'un liens et électrode, d'un clip, d'un shunt, d'un dispositif de fermeture, d'une valvule, d'une particule, d'une microparticule, d'une nanoparticule, d'un gel et d'une émulsion.

7. Le dispositif de la revendication 6, dans lequel le dispositif implantable est une endoprothèse vasculaire.

8. Un dispositif implantable comprenant au moins un agent biologiquement actif choisi dans le groupe constitué des ligands des PPARs, des agonistes des PPARs et des antagonistes des PPARs, dans lequel le dispositif est une endoprothèse vasculaire, une greffe, une endoprothèse vasculaire-greffe, un cathéter, un lien et éléctrode, un clip, un shunt, un dispositif de fermeture, une valvule, une particule, une microparticule, une nanoparticule, un gel et une émulsion, et dans lequel le ligand des PPARs, les agonistes des PPARs et/ou les antagonistes des PPARs sont choisis dans le groupe constitué de la troglitazone, la pioglitazone, et la rosiglitazone.

9. Le dispositif implantable de la revendication 8, dans lequel le dispositif est une endoprothèse vasculaire.
